# EUROPÄISCHE PATENTSCHRIFT

(11) **EP 1 183 015 B1**
(45) Veröffentlichungstag und Bekanntmachung des Hinweises auf die Patenterteilung: **20.10.2004**
(21) Anmeldenummer: 00945739.1
(22) Anmeldetag: 13.06.2000
(51) Int. Cl.: A61K 9/20, A61K 31/19, A61K 31/485

(54) **MEHRSCHICHTTABLETTE ZUR VERABREICHUNG EINER FIXEN KOMBINATION VON TRAMADOL UND DICLOFENAC**
MULTILAYERED TABLET FOR ADMINISTRATION OF A FIXED COMBINATION OF TRAMADOL AND DICLOFENAC
COMPRIME A COUCHES MULTIPLES POUR L'ADMINISTRATION D'UNE COMBINAISON FIXE DE TRAMADOL ET DE DICLOFENAC

(30) Priorität: 17.06.1999 DE 19927688
(43) Veröffentlichungstag der Anmeldung: 06.03.2002
(73) Patentinhaber: Grünenthal GmbH, 52078 Aachen (DE)
(72) Erfinder: BARTHOLOMÄUS, Johannes, D-52080 Aachen (DE); ZIEGLER, Iris, D-52159 Rott-Roetgen (DE)
(74) Vertreter: Kutzenberger, Helga, Dr.
(86) Internationale Anmeldenummer: PCT/EP2000/005385
(87) Internationale Veröffentlichungsnummer: WO 2000/078289

(56) Entgegenhaltungen:
- EP-A- 0 788 790
- EP-A- 0 864 325
- US-A- 4 844 907

## Beschreibung

Die vorliegende Erfindung betrifft eine Mehrschichtablette, welche die Wirkstoffe Tramadol und Diclofenac und/oder deren jeweils physiologisch verträgliche Salze enthält, wobei diese Wirkstoffe durch eine Trennschicht voneinander getrennt sind.

Tramadol stellt ein Analgetikum zur Behandlung starker und mittelstarker Schmerzen dar, dessen Wirkprinzip nicht auf einem reinen Opioid-Mechanismus beruht. Tramadol zeigt nicht die für ein Opioid charakteristischen Nebenwirkungen. in einigen Fällen wird als unerwünschte Begleiterscheinung Übelkeit beobachtet.

Ebenfalls bekannte, nicht opioide, zur Bekämpfung weniger intensiver Schmerzen geeignete Analgetika sind nichtsteroidale Schmerzmittel wie Diclofenac-Na, Acetylsalicylsäure oder lbuprofen.

Desweiteren wird von der WHO für die Behandlung von mäßig bis starken Schmerzen empfohlen, opioide Schmerzmittel mit nicht sterioiden Schmerzmitteln zu kombinieren, um eine effektivere Schmerzlinderung herbeizuführen und gegebenfalls die benötigten Mengen zu verringern.

Das europäische Patent EP -B- 546 676 lehrt zum Beispiel, daß die Kombination von Tramadol-HCl mit steroidfreien entzündungshemmenden Mitteln wie zum Beispiel Ibuprofen in einer Zusammensetzung von 1:1 bis 1:200 zu einer synergistisch verstärkten analgetischen Wirkung führt und die unerwünschten Begleiterscheinungen vermindert. Tramadol-HCl und Diclofenac-Na bilden jedoch eine schwerlösliche Verbindung. Damit ist zu erwarten, daß die Bioverfügbarkeit der beiden Wirkstoffe reduziert ist und zum Ausgleich wesentlich höhere Dosierungen notwendig werden.

Aus der EP 0 788 790 ist eine Tablette mit zeitlich steuerbarer Freisetzung eines pharmazeutischen Wirkstoffes bekannt, welche einen 3-schichtigen Kern umfaßt, der teilweise mit einem Wirkstoff-undurchlässigen Überzug ausgerüstet ist. Die mittlere, ggf. Wirkstoff-haltige Schicht dient zur Einstellung eines Zeitintervalls zwischen der Wirkstofffreisetzung aus den beiden äußeren Schichten.
Eine Tramadol- und Diclofenac-haltige Mehrschichttablette, in der diese Wirkstoffe durch eine Trennschicht voneinander separiert sind, wird in diesem Stand der Technik nicht beschrieben.

Aufgabe der vorliegenden Erfindung war es daher, die beiden Wirkstoffe Tramadol und Diclofenac bzw. deren jeweils physiologisch verträgliche Salze in einer gemeinsamen Darreichungsform zu kombinieren, ohne daß es hierdurch zur Beeinträchtigung der Freisetzungsprofile beider Wirkstoffe und zur Verringerung der Bioverfügbarkeit kommt.

Erfindungsgemäß wird diese Aufgabe durch die Bereitstellung einer oral zu verabreichenden Mehrschichttablette gelöst, die in wenigstens einer Schicht Tramadol und/oder-dessen physiologisch verträgliches Salz und in mindestens einer weiteren Schicht Diclofenac und/oder dessen physiologisch verträgliches Salz enthält, wobei zwischen den wirkstoffhaltigen Schichten jeweils eine Trennschicht vorhanden ist.

Vorzugsweise besteht die Tablette aus sieben Schichten, besonders bevorzugt aus fünf Schichten und ganz besonders bevorzugt aus drei Schichten.

Als physiologisch verträgliche Salze des Tramadols werden vorzugsweise Tramadolhydrochlorid, Tramadolhydrobromid, Tramadolsulfat, Tramadolphosphat, Tramadolfumarat, Tramadolsuccinat, Tramadolmaleat, Tramadolnitrat, Tramadolacetat, Tramadolpropionat, Tramadolmalonat, Tramadolcitrat, Tramadoltartrat, Tramadolbenzoat, Tramadolsalicylat, Tramadolphthalat und/oder Tramadolnicotinat verwendet. Besonders bevorzugt wird Tramadolhydrochlorid verwendet.
Als physiologisch verträgliche Salze des Diclofenacs werden vorzugsweise Diclofenac-Natrium, Diclofenac-Kalium, Diclofenac-Kalzium, Diclofenac-Magnesium und/oder Diclofenac-Colestyramin verwendet. Besonders bevorzugt wird Diclofenac-Natrium verwendet.

Bevorzugt weisen die Tramadol- und/oder die Diclofenac-haltige Wirkstoffschicht der erfindungsgemäßen Mehrschichttablette jeweils eine Schichtdicke von 0,05 mm bis 5 mm, vorzugsweise von 0,1 mm bis 4 mm und besonders bevorzugt von 1 mm bis 3 mm auf.

Die erfindungsgemäß zum Einsatz kommende Mehrschichttablette kann sowohl in der Tramadol- wie auch in der Diclofenac-haltigen Schicht die üblichen Hilfs- und Zusatzstoffe enthalten.

Vorzugsweise enthält die zum Einsatz kommende Mehrschichttablette in einer oder mehreren Schichten jeweils einen der beiden Wirkstoffe in einer retardierenden Matrix, vorzugsweise gleichmäßig verteilt, wodurch optimale, individuell angepaßte Freisetzungswerte erreicht werden können. Durch Kombination mit dem sofort freigesetzten Wirkstoff läßt sich eine Initialdosis zur schnellen Schmerzlinderung erzielen. Die langsame Freisetzung aus der retardierten Form ermöglicht die Aufrechterhaltung therapeutischer Blutspiegel über längere Zeit.

Die Schichten können auch eine retardierte, partikuläre Form des jeweiligen Wirkstoffes enthalten, vorzugsweise Granulate, Mikrokapseln und/oder Pellets, besonders bevorzugt durch Extrusion und/oder Spheronisation hergestellte Pellets. Besonders bevorzugt wird dabei die Freisetzung des Wirkstoffes bzw. der Wirkstoffe so einzustellen sein, daß die Tablette höchstens zweimal, vorzugsweise nur einmal täglich verabreicht werden muß.

Der Anteil beider Analgetika im Verhältnis zu Hilfsstoffen in der Mehrschichttablette wird in Abhängigkeit von der gewünschten Freisetzungsdauer und freizusetzenden Menge der Analgetika eingestellt. Vorzugsweise beträgt der Gehalt an Tramadol 2 bis 60 Gew.%, bevorzugt 5 bis 45 Gew.% und ganz besonders bevorzugt 10 bis 35 Gew.%, bezogen auf das Gesamtgewicht der Mehrschichttablette. Vorzugsweise beträgt der Anteil an Diclofenac 2 bis 30 Gew.%, besonders bevorzugt 5 bis 25 Gew.% und ganz besonders bevorzugt 6 bis 20 Gew. %, bezogen auf das Gesamtgewicht der Mehrschichttablette. Dem Fachmann ist aufgrund der Wirkung der Analgetika bekannt, in welchen Mischungsverhältnissen diese einzusetzen sind, damit die gewünschte Wirkung der Wirkstoffe erreicht wird.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze Amide oder Ester eingesetzt.

Besonders bevorzugt wird als physiologisch verträgliches Material für eine retardierende Matrix mindestens ein Celluloseether und/oder Celluloseester, dessen 2 Gew. % wässrige Lösung bei 20 °C eine Viskosität von 3000 bis 150000 mPas, vorzugsweise von 10000 bis 150000 mPas aufweist, gegebenenfalls in Kombination mit einem im wässrigen Medium nicht quellbaren Füllstoff, wie beispielsweise Calciumhydrogenphosphat, oder mit einem unlöslichen, im wässrigen Medium quellbaren Füllstoff, wie beispielsweise mikrokristalline Cellulose, oder einem in wässrigen Medien löslichen Füllstoff, wie beispielsweise Laktose.

Ebenfalls bevorzugt ist ein Matrixmaterial aus hydrophoben Materialien, wie hydrophobe Polymere, Wachse, Fette, langkettige Fettsäuren, Fettalkohole oder entsprechende Ester oder Ether oder deren Gemische. Besonders bevorzugt werden als hydrophobe Materialien Mono- oder Diglyceride von C12-C30-Fettsäuren und/oder C12-C30-Fettalkohole und/oder Wachse oder deren Gemische eingesetzt.

Bevorzugt kann als Matrixmaterial auch Ethylcellulose, Hydroxyethylcellulose, Hydroxypropylcellulose, Hydroxypropylmethylcellulose, Mono- und Diglyceride von Fettsäuren mit C12-C30 und/oder Fettalkoholen mit C12-C30 oder deren Gemische eingesetzt werden.

Es ist auch möglich, Mischungen der genannten hydrophilen und hydrophoben Materialien als retardierendes Matrixmaterial einzusetzen.

Bevorzugt weist die Trennschicht der erfindungsgemäßen Mehrschichttablette eine Schichtdicke von 0,05 mm bis 10 mm, besonders bevorzugt von 0,1 mm bis 5 mm und ganz besonders bevorzugt von 0,15 mm bis 3 mm auf.

Vorzugsweise besteht die Trennschicht der erfindungsgemäßen Mehrschichttablette aus physiologisch verträglichem Material.

In einer bevorzugten Ausführungsform ist die Trennschicht auch bei Kontakt mit wäßrigen Körperflüssigkeiten für die beiden Wirkstoffe wenig permeabel.
Diese Trennschicht basiert vorzugsweise auf einem Polymeren, einem Wachs, einem Fett, einer Fettsäure, einem Fettalkohol oder einem entsprechenden Ether oder Ester oder einem Gemisch und weist einen Schmelzpunkt von ≥ 40 °C auf.
Als physiologisch verträgliche Polymere werden vorzugsweise Celluloseacetat, Ethylcellulose, Cellulosebutyrat, Polyethylen oder Ethylenvinylacetat-Copolymere verwendet.

In einer weiteren bevorzugten Ausführungsform ist die Trennschicht zwar in Kontakt mit wäßrigen Körperflüssigkeiten für die beiden Wirkstoffe an sich gut permeabel, deren Schichtdicke ist dann aber so zu bemessen, daß die beiden Wirkstoffe während der Dauer der Freisetzung nicht miteinander in Kontakt kommen.

Dazu kann eine permeable Trennschicht der Tablette aus Hydroxyethylcellulose, Hydroxypropylcellulose, Hydroxymethylcellulose und/oder Hydroxypropylmethylcellulose bestehen.

Die permeable Schicht kann ferner auf Celluloseethem, Celluloseestem und/oder Acrylatharzen, vorzugsweise auf Ethylcellulose, Hydroxypropylcellulose, Hydroxymethylcellulose, Hydroxymethylpropylcellulose, Poly(meth)acrylsäure und/oder deren Derivaten, wie deren Salzen, Amiden oder Estern basieren.

Darüber hinaus kann die Trennschicht, wie auch die wirkstoffhaltigen Schichten, weitere Hilfs- und Zusatzstoffe enthalten. Dies können Füllstoffe, vorzugsweise Laktose, mikrokristalline Cellulose oder Calciumhydrogenphosphat, oder Gleit-, Schmier- bzw. Fließregulierungsmittel und/oder Weichmacher, vorzugsweise hochdisperses Siliciumdioxid, Talkum, Magnesiumstearat und/oder Stearinsäure sein.

Die einzelnen Schichten der erfindungsgemäßen Mehrschichttablette können auch so beschaffen sein, daß sie sich bei Kontakt mit wäßrigen Körperflüssigkeiten voneinander lösen und die Wirkstoffe so räumlich getrennt voneinander freisetzen. Als physiologisch verträgliche Sprengmittel, die die Schichten bei Kontakt mit wäßrigen Körperflüssigkeiten voneinander ablösen, können Crospovidon, Croscarmelose, Natriumstärkeglykolat, Stärke und/oder niedrig substituierte Hydroxypropylcellulose verwendet werden.

Ein Gegenstand der Erfindung sind daher Mehrschichttabletten, die mindestens eine Sprengschicht aufweisen, die bei Kontakt mit wäßrigen Körperflüssigkeiten eine Ablösung der verschiedenen Schichten voneinander bewirkt.

Es ist aber auch möglich, daß die Schichten bei Kontakt mit wäßrigen Körperflüssigkeiten miteinander verbunden bleiben und die Wirkstoffe, separiert durch die Trennschicht, unabhängig voneinander freisetzen. Bei der erfindungsgemäßen Mehrschichttablette kann eine kontrollierte Freisetzung beider Wirkstoffe auch durch einen Überzug, der eine kontrollierte, in der Regel retardierte Freisetzung des Wirkstoffes in einem wässrigen Medium gestattet, erreicht werden. Geeignete Retardüberzüge umfassen wasserunlösliche Wachse oder Polymere, wie z.B. Acrylharze, vorzugsweise Poly(meth)acrylate oder wasserunlösliche Cellulosen, wie Ethylcellulose. Diese Materialien sind aus dem Stande der Technik, z.B. Bauer, Lehmann, Osterwald, Rothgang "Überzogene Arzneiformen", Wissenschaftliche Verlagsgesellschaft mbH Stuttgart, 1988, Seite 69 ff., bekannt. Sie werden hiermit als Referenz eingeführt

Neben den wasserunlöslichen Polymeren in dem Überzug können gegebenenfalls zur Einstellung der Freisetzungsrate des Wirkstoffes auch nicht retardierte, vorzugsweise wasserlösliche Polymere in Mengen bis 30 Gew.%, wie Polyvinylpyrrolidon oder wasserlösliche Cellulosen, vorzugsweise Hydroxypropylmethylcellulose oder Hydroxypropylcellulose oder wasserlösliche Porenbildner wie z.B. Lactose, NaCL, Sacchaerose, und/oder die bekannten Weichmacher mitverwendet werden.

Neben dem retardierenden Überzug kann die erfindungsgemäße Mehrschichttablette noch mit weiteren Überzügen ausgerüstet werden. Ein solcher Überzug kann sich zum Beispiel pH-abhängig auflösen. So kann erreicht werden, daß eine bestimmte Wirkstoffmenge den Magentrakt unaufgelöst passiert und erst im Darmtrakt zur Freisetzung gelangt.

Die erfindungsgemäße Mehrschichttablette kann außerdem mindestens eine Bruchkerbe aufweisen, die es ermöglicht die einzunehmende Dosis aufzuteilen, vorzugsweise zu halbieren. Dies ermöglicht eine Anpassung an die individuellen Bedürfnisse des Patienten, entsprechend der individuell zu verabreichenden Menge der Analgetika.

Ein weiterer Gegenstand der Erfindung sind daher Mehrschichttabletten, die mindestens eine Bruchkerbe aufweisen, welche die Aufteilung der Dosis, vorzugsweise deren Halbierung, ermöglicht.

Die Herstellung der Mehrschichttabletten erfolgt nach bekannten Methoden, wie sie z.B in R. Voigt, "Lehrbuch der pharmazeutischen Technologie", 6. Auflage, Seite 225 ff. beschrieben sind. Sie werden hiermit als Referenz eingeführt.

Vorzugsweise erfolgt die Herstellung der Mehrschichttabletten, indem die Bestandteile der einzelnen Schichten zunächst getrennt durch Mischen der einzelnen Bestandteile in einem Mischer und gegebenfalls Granulation vermengt werden. Die verschiedenen Schichten werden dann auf einer Tablettenpresse, vorzugsweise einer Rundläufermehrschichttablettenpresse, aufeinanderfolgend so zu einer Tablette verpreßt, daß durch die Trennschicht die wirkstoffhaltigen Schichten nicht miteinander in Kontakt kommen.

Die erfindungsgemäßen Mehrschichttabletten können die üblichen Formen und Größen aufweisen.
Sofern die erfindungsgemäße Mehrschichttablette Überzüge enthält, können diese nach üblichen Verfahren, wie z.B. Dragieren, Aufsprühen von Lösungen, Dispersionen oder Suspensionen, durch Schmelzverfahren oder durch Pulverauftragsverfahren aufgebracht werden.

Sofern die erfindungsgemäße Mehrschichttablette für eine zweimalige Applikation pro Tag vorgesehen ist, wird das Freisetzungsprofil für den Wirkstoff Tramadol aus der erfindungsgemäßen Mehrschichttablette vorzugsweise so gesteuert, daß die freigesetzte Menge an Tramadol in % bezogen auf die Gesamtmenge nach 30 Minuten 2 bis 40 %, bevorzugt 5 bis 30 %, nach 120 Minuten 5 bis 80 %, bevorzugt 20 bis 60, nach 240 Minuten 30 bis 90 %, bevorzugt 35 bis 75 %, nach 360 Minuten 50 bis 95 %, bevorzugt 60 bis 90 %, nach 480 Minuten 60 bis 100 %, bevorzugt 70 bis 100 % und nach 600 Minuten 70 bis 100 %, bevorzugt 75 bis 100 % beträgt. Das Freisetzungsprofil für den Wirkstoff Tramadol für eine einmalige Applikation pro Tag aus der erfindungsgemäßen Mehrschichttablette ist vorzugsweise so gesteuert, daß die freigesetzte Menge an Tramadol in % bezogen auf die Gesamtmenge nach 60 Minuten 2 bis 40 %, bevorzugt 5 bis 30 %, nach 240 Minuten 5 bis 80 %, bevorzugt 20 bis 60, nach 480 Minuten 30 bis 90 %, bevorzugt 35 bis 75 %, nach 720 Minuten 50 bis 95 %, bevorzugt 60 bis 90 %, nach 960 Minuten 60 bis 100 %, bevorzugt 70 bis 100 % und nach 1200 Minuten 70 bis 100 %, bevorzugt 75 bis 100 % beträgt.

Das Freisetzungsprofil für den Wirkstoff Diclofenac für eine zweimalige Applikation pro Tag aus der erfindungsgemäßen Mehrschichttablette ist vorzugsweise so gesteuert, daß die freigesetzte Menge an Diclofenac in % bezogen auf die Gesamtmenge nach 30 Minuten 0 %, nach 120 Minuten ≤ 5 %, nach 240 Minuten 5 bis 50 %, bevorzugt 10 bis 35 %, 480 Minuten 30 bis 95 %, bevorzugt 35 bis 80 % und nach 600 Minuten 45 bis 100 %, bevorzugt 60 bis 100 % beträgt.
Das Freisetzungsprofil für den Wirkstoff Diclofenac für eine einmalige Applikation pro Tag aus der erfindungsgemäßen Mehrschichttablette ist vorzugsweise so gesteuert, daß die freigesetzte Menge an Diclofenac in % bezogen auf die Gesamtmenge nach 120 Minuten ≤ 5 %, nach 240 Minuten 5 bis 40 %, bevorzugt 10 bis 30 %, nach 480 Minuten 15 bis 60 %, bevorzugt 15 bis 50 %, nach 720 Minuten 30 bis 80 %, bevorzugt 35 bis 80 % und nach 1200 Minuten 50 bis 100 %, bevorzugt 60 bis 100 % beträgt.

Gegenstand der Erfindung sind daher Mehrschichttabletten, die dadurch gekennzeichnet sind, daß bei deren zweimaligen Applikation pro Tag jeweils innerhalb von 8 Stunden ≥ 70 % des Tramadol und ≥ 60 % des Diclofenac freigesetzt werden.
Ein weiterer Gegenstand der Erfindung sind auch Mehrschichttabletten, die dadurch gekennzeichnet sind, daß bei deren einmaligen Applikation pro Tag innerhalb von 16 Stunden ≥ 70 % des Tramadol und ≥ 60 % des Diclofenac freigesetzt werden.

Die Freisetzungsprofile wurden gemäß der nachstehende Methode bestimmt.

Bei magensaftresistenten Tabletten ist den genannten Freisetzungsprofilen bezüglich der Zeiten des Tramadols außerdem die Verweilzeit im Magen hinzuzurechnen.

Die Freisetzungsprofile der erfindungsgemäßen Mehrschichttabletten wurde wie folgt bestimmmt:

Die erfindungsgemäßen Tabletten wurden in der Freisetzungsapparatur mit Blattrührer gemäß Pharm. Eur. bei einer Temperatur des Freisetzungsmediums von 37°C und einer Umdrehungsgeschwindigkeit des Blattrührers von 75 min-1, 2 Stunden lang in 600 ml künstlichem Magensaft ohne Enzyme (pH 1,2) gegeben. Anschließend wurden die Zubereitungen weitere 8 Stunden lang in 900 ml künstlichem Darmsaft ohne Enzyme (pH 7,2) gegeben. Die jeweils zu einem Zeitpunkt freigesetzte Menge des jeweiligen Wirkstoffes Tramadol oder Diclofenac wird durch HPLC bestimmt. Die dargestellten Werte und Kurven sind die Mittelwerte aus jeweils 6 Proben.
Die folgenden Beispiele dienen der Erläuterung der Erfindung.

### Beispiel 1:

Die Schichten der erfindungsgemäßen Mehrschichttablette wurden zunächst einzeln hergestellt. Hierzu wurde die Tramadolhydrochlorid enthaltende Wirkstoffschicht durch Mischen von Tramadolhydrochlorid, mikrokristalliner Cellulose, Hydroxypropylmethylcellulose, hochdispersem Siliciumdioxid und Magnesiumstearat in einem Kubusmischer zubereitet. Die Trennschicht wurde durch Mischen von mikrokristalliner Cellulose, Hydroxypropylmethylcellulose, hochdispersem Siliciumdioxid und Magnesiumstearat in einem Kubusmischer zubereitet. Die Diclofenac-Na enthaltende Wirkstoffschicht wurde durch Mischen von mikronisiertem Diclofenac-Na, mikrokristalliner Cellulose, Hydroxypropymethylcellulose, hochdispersem Siliciumdioxid und Magnesiumstearat in einem Kubusmischer zubereitet.
Anschließend wurden die beiden wirkstoffhaltigen Schichten mit der dazwischen liegenden Trennschicht in einem Arbeitsgang zu einer 3-Schichttablette mit einem Durchmesser von 12 mm verpreßt. Dazu wurden jeweils die Menge einer Schicht aufeinanderfolgend in einer Matrize mittels einer Exzentertablettenpresse leicht verpreßt und anschließend die gesamte Schichtfolge verpreßt. Die Tablette wies eine Härte von 100 bis 130 N gemäß Erweka Bruchfestigkeitstester auf.

| Zusammensetzung der 3-Schichttablette | | |
|---|---|---|
| Tramadolhydrochlorid | 100,00 mg | |
| Mikrokristalline Cellulose (Avicel PH 101; FMC) | 82,00 mg | |
| Hydroxypropylmethylcellulose, 100 000 mPa · s (Metolose 90 SH 100 000, ShinEtsu) | 63,00 mg | |
| Hochdisperses Siliciumdioxid (Aerosil, Degussa) | 2,50 mg | |
| Magnesiumstearat | 2,50 mg | **1. Schicht: 250 mg** |
| Mikrokristalline Cellulose (Avicel PH 101, FMC) | 71,00 mg | |
| Hydroxypropylmethylcellulose, 100 000 mPa · s (Metolose 90 SH 100 000, ShinEtsu) | 27,00 mg | |
| Hochdisperses Siliciumdioxid (Aerosil, Degussa) | 1,00 mg | |
| Magnesiumstearat | 1,00 mg | **Trennschicht: 100 mg** |
| Diclofenac-Na, mikronisiert | 50,00 mg | |
| Mikrokristalline Cellulose (Avicel PH 101, FMC) | 132,00 mg | |
| Hydroxypropylmethylcellulose, 100 000 mPa · s (Metolose 90 SH 100 000, ShinEtsu) | 63,00 mg | |
| Hochdisperses Siliciumdioxid (Aerosil, Degussa) | 2,50 mg | |
| Magnesiumstearat | 2,50 mg | **3. Schicht: 250 mg** |
| **Gesamt** | **600,00 mg** | |

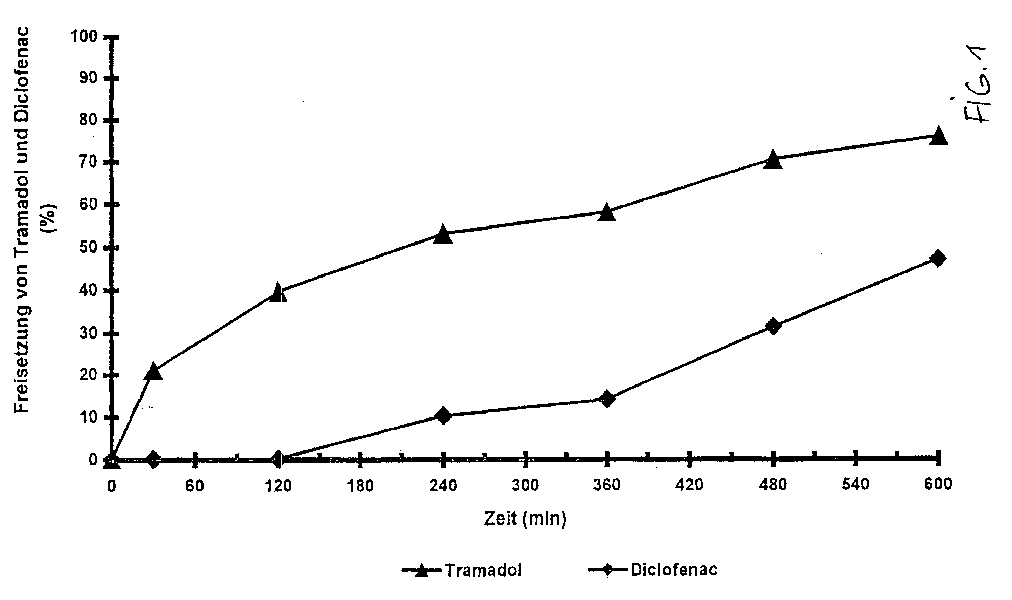

Das Freisetzungsprofil war wie folgt und ist in Abb. 1 dargestellt:

| Zeit in min | Freigesetzter Anteil in % | |
|---|---|---|
| | Diclofenac | Tramadol |
| 30 | 0 | 21 |
| 120 | 0 | 40 |
| 240 | 11 | 54 |
| 360 | 14 | 59 |
| 480 | 32 | 72 |
| 600 | 48 | 77 |

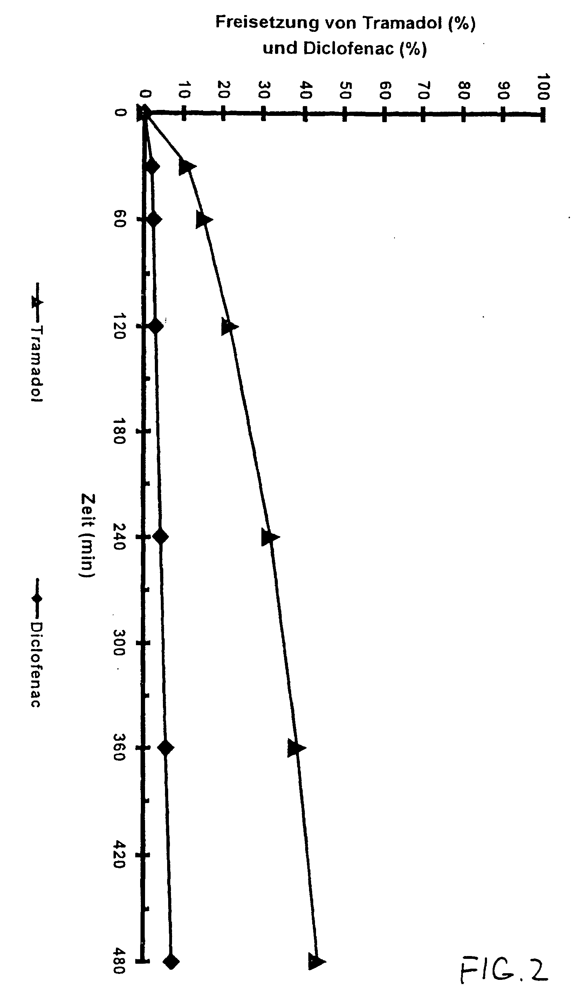

Die Abb. 2 zeigt das Freisetzungsprofil einer Matrixtablette mit einem Durchmesser von 12 mm, die 100 mg Tramadol und 50 mg Diclofenac gemeinsam verpreßt in einer hydrophilen Matrix aus Hydroxypropylmethylcellulose enthält. Ein Vergleich der Abb. 1 mit Abb. 2 zeigt, daß die freigesetzte Menge der Wirkstoffe Tramadol und Diclofenac aus der erfindungsgemäßen 3-Schichttablette nach 8 Stunden deutlich größer ist als die Freisetzung aus den sogenannten gemeinsamen Matrixtabletten.

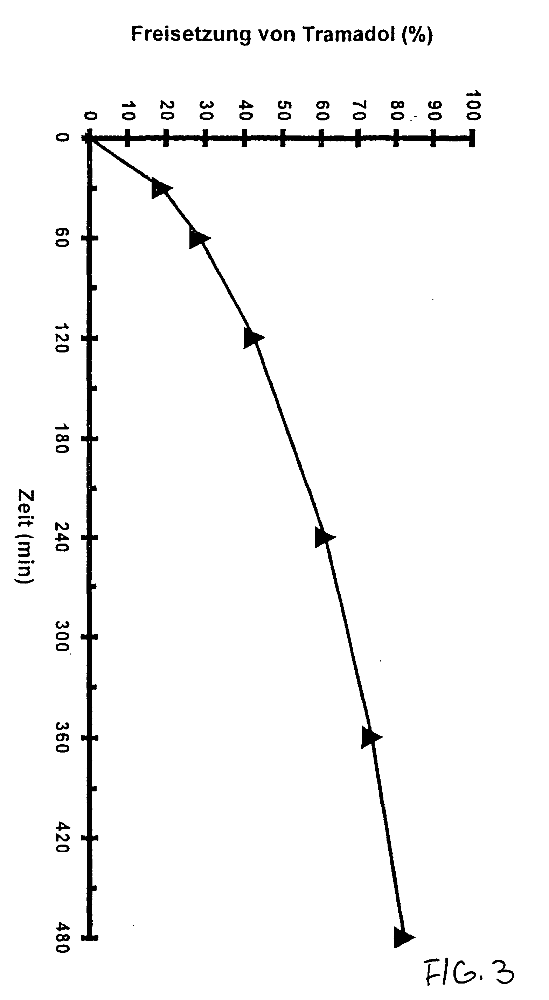

Die Abb. 3 zeigt das Freisetzungsprofil von retardierten Matrixtabletten mit einem Durchmesser von 10 mm, die als Wirkstoff nur 100 mg Tramadol in einer hydrophilen Matrix aus Hydroxypropylmethylcellulose enthalten. Ein Vergleich der Abb. 1 mit Abb. 3 zeigt, daß die freigesetzten Mengen an Tramadol aus den erfindungsgemäßen 3-Schichttabletten mit ≥ 75 % der Freisetzung aus den Tramadol Tabletten mit ≥ 80 % entsprechen.

### Beispiel 2

Die Zubereitung der einzelnen Schichten erfolgte analog zu Beispiel 1. Anschließend wurden die beiden wirkstoffhaltigen Schichten mit der dazwischen liegenden Trennschicht analog zu einer 3-Schichttablette mit einem Durchmesser von 16 mm verpreßt.

| Zusammensetzung der 3-Schichttablette | | |
|---|---|---|
| Tramadolhydrochlorid | 100.00 mg | |
| Mikrokristalline Cellulose (Avicel PH 101; FMC) | 82.00 mg | |
| Hydroxypropylmethylcellulose, 100 000 mPa · s (Metolose 90 SH 100 000, ShinEtsu) | 63.00 mg | |
| Hochdisperses Siliciumdioxid (Aerosil, Degussa) | 2.50 mg | |
| Magnesiumstearat | 2.50 mg | **1. Schicht: 250 mg** |
| Mikrokristalline Cellulose (Avicel PH 101, FMC) | 94.00 mg | |
| Quervernetztes Polyvinylpyrolidon (Kollidon CL, BASF) | 5.00 mg | |
| Magnesiumstearat | 1.00 mg | **Trennschicht: 100 mg** |
| Diclofenac-Na, mikronisiert | 50.00 mg | |
| Mikrokristalline Cellulose (Avicel PH 101, FMC) | 132.00 mg | |
| Hydroxypropylmethylcellulose, 100 000 mPa s (Metolose 90 SH 100 000, ShinEtsu) | 63.00 mg | |
| Hochdisperses Siliciumdioxid (Aerosil, Degussa) | 2.50 mg | |
| Magnesiumstearat | 2.50 mg | **3. Schicht: 250 mg** |
| **Gesamt** | **600.00 mg** | |

Das Freisetzungsprofil war wie folgt:

| Zeit in min | Freigesetzter Anteil in % | |
|---|---|---|
| | Diclofenac | Tramadol |
| 30 | 0 | 35 |
| 120 | 0 | 71 |
| 240 | 14 | 85 |
| 360 | 18 | 90 |
| 480 | 37 | 95 |
| 600 | 56 | 99 |

### Beispiel 3

Tramadolhydrochlorid und Diclofenac-Na wurden analog Beispiel 1 jeweils mit mikrokristalliner Cellulose, Hydroxypropylmethylcellulose, hochdispersem Siliciumdioxid und Magnesiumstearat in einem geeignetem Mischer gemischt und anschließend auf einer geeigneten Tablettenpresse gemeinsam mit einer wirkstofffreien Zwischenschicht aus Mikrokristalliner Cellulose, quervernetztem Polyvinylpyrolidon und Magnesiumstearat zu 3-Schichttabletten mit den Maßen 7 mm x 14 mm mit einer Bruchkerbe verpreßt. Die Tabletten besitzen eine Härte von 100 - 130 N.

| Zusammensetzung der 3-Schichttablette | | |
|---|---|---|
| Tramadolhydrochlorid | 100.00 mg | |
| Mikrokristalline Cellulose (Avicel PH 101; FMC) | 82.00 mg | |
| Hydroxypropylmethylcellulose, 100 000 mPa · s (Metolose 90 SH 100 000, ShinEtsu) | 63.00 mg | |
| Hochdisperses Siliciumdioxid (Aerosil, Degussa) | 2.50 mg | |
| Magnesiumstearat | 2.50 mg | **1. Schicht: 250 mg** |
| Mikrokristalline Cellulose (Avicel PH 101, FMC) | 94.00 mg | |
| Quervernetztes Polyvinylpyrolidon (Kollidon CL, BASF) | 5.00 mg | |
| Magnesiumstearat | 1.00 mg | **Trennschicht: 100 mg** |
| Diclofenac-Na, mikronisiert | 50.00 mg | |
| Mikrokristalline Cellulose (Avicel PH 101, FMC) | 132.00 mg | |
| Hydroxypropylmethylcellulose, 100 000 mPa s (Metolose 90 SH 100 000, ShinEtsu) | 63.00 mg | |
| Hochdisperses Siliciumdioxid (Aerosil, Degussa) | 2.50 mg | |
| Magnesiumstearat | 2.50 mg | **3. Schicht: 250 mg** |
| **Gesamt** | **600.00 mg** | |

Das Freisetzungsprofil wurde analog Beispiel 1 bestimmt und ist in der nachfolgenden Tabelle angegeben, wobei sich die Einzelschichten der Tablette im Verlauf der ersten Stunden der Freisetzung voneinander ablösen und dann jeweils als separate Einheiten in der Freisetzungsapparatur vorliegen und freisetzen.

| Zeit in min | Freigesetzter | Anteil in % |
|---|---|---|
| | Diclofenac | Tramadol |
| 30 | 0 | 22 |
| 120 | 0 | 45 |
| 240 | 15 | 60 |
| 360 | 31 | 70 |
| 480 | 50 | 80 |
| 600 | 72 | 96 |

## Patentansprüche

1. Mehrschichttablette enthaltend in mindestens einer Schicht Tramadol und in mindestens einer Schicht Diclofenac und/oder deren jeweils physiologisch verträgliches Salz, wobei diese Wirkstoffe Tramadol und Diclofenac jeweils durch eine Trennschicht voneinander getrennt sind.

2. Mehrschichttablette nach Anspruch 1, **dadurch gekennzeichnet, daß** sie als physiologisch verträgliches Salz des Tramadols Tramadolhydrochlorid, Tramadolhydrobromid, Tramadolsulfat, Tramadolphosphat, Tramadolfumarat, Tramadolsuccinat, Tramadolmaleat, Tramadolnitrat, Tramadolacetat, Tramadolpropionat, Tramadolmalonat, Tramadolcitrat, Tramadoltartrat, Tramadolbenzoat, Tramadolsalicylat, Tramadolphthalat und/oder Tramadolnicotinat, besonders bevorzugt Tramadolhydrochlorid und als physiologisch verträgliches Salz des Diclofenacs Diclofenac-Natrium, Diclofenac-Kalium, Diclofenac-Kalzium, Diclofenac-Magnesium und/oder Diclofenac-Colestyramin, bevorzugt Diclofenac-Natrium enthält.

3. Mehrschichttablette nach Anspruch 1 oder 2, **dadurch gekennzeichnet, daß** sie aus sieben Schichten, bevorzugt aus fünf Schichten und besonders bevorzugt aus drei Schichten besteht.

4. Mehrschichttablette nach einem oder mehreren der Ansprüche 1 bis 3, **dadurch gekennzeichnet, daß** der Gehalt an Tramadol-HCl 2 bis 60 Gew. %, bevorzugt 5 bis 45 Gew. % und besonders bevorzugt 10 bis 35 Gew. % bezogen auf das Gesamtgewicht der Mehrschichttablette beträgt.

5. Mehrschichttablette nach einem oder mehreren der Ansprüche 1 bis 4, **dadurch gekennzeichnet, daß** der Gehalt an Diclofenac-Na 2 bis 30 Gew. %, bevorzugt 5 bis 25 Gew. % und besonders bevorzugt 6 bis 20 Gew. %, bezogen auf das Gesamtgewicht der Mehrschichttablette beträgt.

6. Mehrschichttablette nach einem oder mehreren der Ansprüche 1 bis 5, **dadurch gekennzeichnet, daß** die Tramadol- und/oder die Diclofenac-haltige Wirkstoffschicht jeweils eine Schichtdicke von 0,05 mm bis 5 mm, vorzugsweise von 0,1 mm bis 4 mm und besonders bevorzugt von 1 mm bis 3 mm aufweist.

7. Mehrschichttablette nach einem oder mehreren der Ansprüche 1 bis 6, **dadurch gekennzeichnet, daß** wenigstens eine der wirkstoffhaltigen Schichten den Wirkstoff in einer retardierenden Matrix, vorzugsweise gleichmäßig verteilt, aufweist.

8. Mehrschichttablette nach Anspruch 7, **dadurch gekennzeichnet, daß** die Matrix wenigstens ein Polymeres, ein Wachs, ein Fett, eine Fettsäure, einen Fettalkohol oder einen entsprechenden Ester oder Ether aufweist.

9. Mehrschichttablette nach Anspruch 8, **dadurch gekennzeichnet, daß** als Polymere Celluloseether, Celluloseester und/oder Acrylharze eingesetzt werden.

10. Mehrschichttablette nach Anspruch 9, **dadurch gekennzeichnet, daß** Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt werden.

11. Mehrschichttablette nach einem oder mehreren der Ansprüche 7 bis 9, **dadurch gekennzeichnet, daß** als Matrixmaterial Ethylcellulose, Hydroxyethylcellulose, Hydroxypropylcellulose, Hydroxypropylmethylcellulose, Mono- und Diglyceride von Fettsäuren mit C12-C30 und/oder Fettalkoholen mit C12-C30 oder deren Gemische eingesetzt werden.

12. Mehrschichttablette nach einem oder mehreren der Ansprüche 1 bis 11, **dadurch gekennzeichnet, daß** mindestens eine Schicht eine retardierte, partikuläre Form von Tramadol oder Diclofenac und/oder deren entsprechende physiologisch verträgliche Salze enthält, bevorzugt als Granulate, Mikrokapseln und/oder Pellets, besonders bevorzugt durch Extrusion und/oder Spheronisation hergestellte Pellets.

13. Mehrschichttablette nach einem oder mehreren der Ansprüche 1 bis 12, **dadurch gekennzeichnet, daß** die Trennschicht eine Schichtdicke von 0,05 mm bis 10 mm, vorzugsweise von 0,1 mm bis 5 mm und besonders bevorzugt von 0,15 mm bis 3 mm aufweist.

14. Mehrschichttablette nach einem oder mehreren der Ansprüche 1 bis 13, **dadurch gekennzeichnet, daß** die Trennschicht aus physiologisch verträglichem Material besteht.

15. Mehrschichttablette nach einem oder mehreren der Ansprüche 1 bis 14, **dadurch gekennzeichnet, daß** die Trennschicht auf einem, für die Wirkstoffe nicht oder wenig permeablem, physiologisch verträglichem Material, vorzugsweise einem Polymeren, einem Wachs, einem Fett, einer Fettsäure, einem Fettalkohol oder einem entsprechenden Ether oder Ester basiert und einen Schmelzpunkt von ≥ 40 °C aufweist.

16. Mehrschichttablette nach Anspruch 15, **dadurch gekennzeichnet, daß** die Trennschicht auf einem physiologisch verträglichen Polymeren, vorzugsweise Celluloseacetat, Ethylcellulose, Cellulosebutyrat, Polyethylen oder Ethylenvinylacetat-Copolymeren basiert.

17. Mehrschichttablette nach einem oder mehreren der Ansprüche 1 bis 14, **dadurch gekennzeichnet, daß** die Trennschicht zwar aus für die Wirkstoffe an sich permeablem Material besteht, die Schichtdicke aber so bemessen ist, daß die beiden Wirkstoffe während der Freisetzungsdauer nicht miteinander in Kontakt kommen.

18. Mehrschichttablette nach Anspruch 17, **dadurch gekennzeichnet, daß** die permeable Schicht auf Celluloseethern, Celluloseestern und/oder Acrylatharzen, vorzugsweise auf Ethylcellulose, Hydroxypropylcellulose, Hydroxymethylcellulose, Hydroxymethylpropylcellulose, Poly(meth)acrylsäure und/oder deren Derivaten, wie deren Salzen, Amiden oder Estern basiert.

19. Mehrschichttablette gemäß Anspruch 17, **dadurch gekennzeichnet, daß** die permeable Trennschicht aus Hydroxyethylcellulose, Hydroxypropylcellulose, Hydroxymethylcellulose und/oder Hydroxypropylmethylcellulose besteht.

20. Mehrschichttablette nach einem oder mehreren der Ansprüche 1 bis 19, **dadurch gekennzeichnet, daß** die Trennschicht und/oder die wirkstoffhaltigen Schichten weitere Hilfsstoffe, wie Füllstoffe, Gleit-, Schmier- und Fließregulierungsmittel enthält.

21. Mehrschichttablette nach einem oder mehreren der Ansprüche 1 bis 20, **dadurch gekennzeichnet, daß** sie mit mindestens einem Überzug versehen ist.

22. Mehrschichttablette nach Anspruch 21, **dadurch gekennzeichnet, daß** der Überzug retardierend ist.

23. Mehrschichttablette nach Anspruch 22, **dadurch gekennzeichnet, daß** der Überzug auf einem wasserunlöslichen Polymeren oder Wachs basiert.

24. Mehrschichttablette nach Anspruch 23, **dadurch gekennzeichnet, daß** als Polymeres ein Polyacrylharz oder Cellulosederivat, vorzugsweise Alkylcellulose, eingesetzt wird.

25. Mehrschichttablette nach Anspruch 24, **dadurch gekennzeichnet, daß** Ethylcellulose und/oder ein Poly(meth)acrylat als Polymeres eingesetzt wird.

26. Mehrschichttablette zur zweimaligen Applikation pro Tag nach einem der Ansprüche 1 bis 25, **dadurch gekennzeichnet, daß** das Tramadol bzw. das Diclofenac innerhalb von 8 Stunden zu ≥ 70 Gew.% bzw. zu ≥ 60 Gew.% freigesetzt wird.

27. Mehrschichttablette zur einmaligen Applikation pro Tag nach einem oder mehreren der Ansprüche 1 bis 26, **dadurch gekennzeichnet, daß** das Tramadol bzw. das Diclofenac innerhalb von 16 Stunden zu ≥ 70 Gew.% bzw. zu ≥ 60 Gew.% freigesetzt wird.

28. Mehrschichttablette nach einem oder mehreren der Ansprüche 1 bis 27, **dadurch gekennzeichnet, daß** sie mindestens eine Sprengschicht aufweist, die bei Kontakt mit wäßrigen Körperflüssigkeiten die Ablösung der verschiedenen Schichten voneinander bewirkt.

29. Mehrschichttablette nach einem oder mehreren der Ansprüche 1 bis 28, **dadurch gekennzeichnet, daß** die Tablette mindestens eine Bruchkerbe enthält, welche die Teilung der zu verabreichenden Dosis, vorzugsweise die Halbierung, ermöglicht.

## Claims

1. A multilayer tablet containing tramadol in at least one layer and diclofenac in at least one layer and/or in each case the physiologically acceptable salt thereof, wherein these active ingredients tramadol and diclofenac are in each case separated from one another by a separation layer.

2. A multilayer tablet according to claim 1, **characterised in that** the physiologically acceptable salt of tramadol it contains is tramadol hydrochloride, tramadol hydrobromide, tramadol sulfate, tramadol phosphate, tramadol fumarate, tramadol succinate, tramadol maleate, tramadol nitrate, tramadol acetate, tramadol propionate, tramadol malonate, tramadol citrate, tramadol tartrate, tramadol benzoate, tramadol salicylate, tramadol phthalate and/or tramadol nicotinate, particularly preferably tramadol hydrochloride and the physiologically acceptable salt of diclofenac it contains is diclofenac sodium, diclofenac potassium, diclofenac calcium, diclofenac magnesium and/or diclofenac colestyramine, preferably diclofenac sodium.

3. A multilayer tablet according to claim 1 or 2, **characterised in that** it consists of seven layers, preferably of five layers and particularly preferably of three layers.

4. A multilayer tablet according to one or more of claims 1 to 3, **characterised in that** the content of tramadol HCl is 2 to 60 wt.%, preferably 5 to 45 wt.% and particularly preferably 10 to 35 wt.% relative to the total weight of the multilayer tablet.

5. A multilayer tablet according to one or more of claims 1 to 4, **characterised in that** the content of diclofenac sodium is 2 to 30 wt.%, preferably 5 to 25 wt.% and particularly preferably 6 to 20 wt.%, relative to the total weight of the multilayer tablet.

6. A multilayer tablet according to one or more of claims 1 to 5, **characterised in that** the active ingredient layer containing tramadol and/or the active ingredient layer containing diclofenac in each case exhibits a layer thickness of 0.05 mm to 5 mm, preferably of 0.1 mm to 4 mm and particularly preferably of 1 mm to 3 mm.

7. A multilayer tablet according to one or more of claims 1 to 6, **characterised in that** at least one of the active ingredient containing layers comprises the active ingredient, preferably uniformly distributed, in a delayed-release matrix.

8. A multilayer tablet according to claim 7, **characterised in that** the matrix comprises at least one polymer, a wax, a fat, a fatty acid, a fatty alcohol or a corresponding ester or ether.

9. A multilayer tablet according to claim 8, **characterised in that** cellulose ethers, cellulose esters and/or acrylic resins are used as the polymer.

10. A multilayer tablet according to claim 9, **characterised in that** ethylcellulose, hydroxypropylmethylcellulose, hydroxypropylcellulose, hydroxymethylcellulose, poly(meth)acrylic acid and/or the derivatives thereof, such as the salts, amides or esters thereof, are used.

11. A multilayer tablet according to one or more of claims 7 to 9, **characterised in that** the matrix material used is ethylcellulose, hydroxyethylcellulose, hydroxypropylcellulose, hydroxypropylmethylcellulose, mono- and diglycerides of C12-C30 fatty acids and/or C12-C30 fatty alcohols or mixtures thereof.

12. A multilayer tablet according to one or more of claims 1 to 11, **characterised in that** at least one layer contains a delayed-release, particulate form of tramadol or diclofenac and/or the corresponding physiologically acceptable salts thereof, preferably as granules, microcapsules and/or pellets, particularly preferably pellets produced by extrusion and/or spheronisation.

13. A multilayer tablet according to one or more of claims 1 to 12, **characterised in that** the separation layer has a layer thickness of 0.05 mm to 10 mm, preferably of 0.1 mm to 5 mm and particularly preferably of 0.15 mm to 3 mm.

14. A multilayer tablet according to one or more of claims 1 to 13, **characterised in that** the separation layer consists of physiologically acceptable material.

15. A multilayer tablet according to one or more of claims 1 to 14, **characterised in that** the separation layer is based on a physiologically acceptable material which is impermeable or sparingly permeable to the active ingredients, preferably a polymer, a wax, a fat, a fatty acid, a fatty alcohol or a corresponding ether or ester and has a melting point of ≥ 40°C.

16. A multilayer tablet according to claim 15, **characterised in that** the separation layer is based on a physiologically acceptable polymer, preferably cellulose acetate, ethylcellulose, cellulose butyrate, polyethylene or ethylene/vinyl acetate copolymers.

17. A multilayer tablet according to one or more of claims 1 to 14, **characterised in that**, while the separation layer does indeed consist of a material which is per se permeable to the active ingredients, the layer thickness is calculated such that the two active ingredients do not come into contact with one another during the duration of release.

18. A multilayer tablet according to claim 17, **characterised in that** the permeable layer is based on cellulose ethers, cellulose esters and/or acrylate resins, preferably on ethylcellulose, hydroxypropylcellulose, hydroxymethylcellulose, hydroxymethylpropylcellulose, poly(meth)acrylic acid and/or derivatives thereof, such as the salts, amides or esters thereof.

19. A multilayer tablet according to claim 17, **characterised in that** the permeable separation layer consists of hydroxyethylcellulose, hydroxypropylcellulose, hydroxymethylcellulose and/or hydroxypropylmethylcellulose.

20. A multilayer tablet according to one or more of claims 1 to 19, **characterised in that** the separation layer and/or the layers containing active ingredient contain further auxiliary substances, such as fillers, slip, lubricant and flow-control agents.

21. A multilayer tablet according to one or more of claims 1 to 20, **characterised in that** it is provided with at least one coating.

22. A multilayer tablet according to claim 21, **characterised in that** the coating delays release.

23. A multilayer tablet according to claim 22, **characterised in that** the coating is based on a water-insoluble polymer or wax.

24. A multilayer tablet according to claim 23, **characterised in that** a polyacrylic resin or cellulose derivative, preferably alkylcellulose, is used as the polymer.

25. A multilayer tablet according to claim 24, **characterised in that** ethylcellulose and/or a poly(meth)acrylate is used as the polymer.

26. A multilayer tablet for twice daily administration according to one of claims 1 to 25, **characterised in that** the tramadol and the diclofenac are respectively ≥ 70 wt.% and ≥ 60 wt.% released within 8 hours.

27. A multilayer tablet for once daily administration according to one or more of claims 1 to 26, **characterised in that** the tramadol and the diclofenac are respectively ≥ 70 wt.% and ≥ 60 wt.% released within 16 hours.

28. A multilayer tablet according to one or more of claims 1 to 27, **characterised in that** it comprises at least one disintegrant layer which, on contact with aqueous bodily fluids, causes the various layers to become detached from one another.

29. A multilayer tablet according to one or more of claims 1 to 28, **characterised in that** the tablet contains at least one notch which enables division, preferably halving, of the dose to be administered.

## Revendications

1. Comprimé multicouche contenant au moins une couche de Tramadol et au moins une couche de Diclofénac et/ou à chaque fois leur sel physiologiquement compatible, ces principes actifs Tramadol et Diclofénac étant à chaque fois séparés l'un de l'autre par une couche de séparation.

2. Comprimé multicouche selon la revendication 1, **caractérisé en ce qu'**il contient comme sel de Tramadol physiologiquement compatible le chlorhydrate de Tramadol, l'hydrobromure de Tramadol, le sulfate de Tramadol, le phosphate de Tramadol, le fumarate de Tramadol, le succinate de Tramadol, le maléate de Tramadol, le nitrate de Tramadol, l'acétate de Tramadol, le propionate de Tramadol, le malonate de Tramadol, le citrate de Tramadol, le tartrate de Tramadol, le benzoate de Tramadol, le salicylate de Tramadol, le phtalate de Tramadol et/ou le nicotinate de Tramadol, et de manière particulièrement préférée, le chlorhydrate de Tramadol et comme sel de Diclofénac physiologiquement compatible le sodium de Diclofénac, le potassium de Diclofénac, le calcium de Diclofénac, le magnésium de Diclofénac et/ou la coléstyramine de Diclofénac, de préférence le sodium de Diclofénac.

3. Comprimé multicouche selon la revendication 1 ou 2, **caractérisé en ce qu'**il se compose de sept couches, de préférence de cinq couches et de manière particulièrement préférée de trois couches.

4. Comprimé multicouche selon l'une quelconque ou plusieurs des revendications 1 à 3, **caractérisé en ce que** la teneur en HCl de Tramadol s'élève de 2 à 60 % en poids, de préférence de 5 à 45 % en poids et de manière particulièrement préférée de 10 à 35 % en poids, rapporté au poids total du comprimé multicouche.

5. Comprimé multicouche selon l'une quelconque ou plusieurs des revendications 1 à 4, **caractérisé en ce que** la teneur en sodium de Diclofénac s'élève de 2 à 30 % en poids, de préférence de 5 à 25 % en poids et de manière particulièrement préférée de 6 à 20 % en poids, rapporté au poids total du comprimé multicouche.

6. Comprimé multicouche selon l'une quelconque ou plusieurs des revendications 1 à 5, **caractérisé en ce que** la couche de principe actif contenant le Tramadol et/ou celle contenant le Diclofénac présentent à chaque fois une épaisseur de couche de 0,05 mm à 5 mm, de préférence de 0,1 mm à 4 mm et de manière particulièrement préférée de 1 mm à 3 mm.

7. Comprimé multicouche selon l'une quelconque ou plusieurs des revendications 1 à 6, **caractérisé en ce qu'**au moins une des couches contenant le principe actif présente le principe actif dans une matrice à retardement, de préférence réparti de manière homogène.

8. Comprimé multicouche selon la revendication 7, **caractérisé en ce que** la matrice présente au moins un polymère, une cire, une matière grasse, un acide gras, un alcool gras ou un ester ou éther correspondant.

9. Comprimé multicouche selon la revendication 8, **caractérisé en ce que** comme polymères on utilise un éther de cellulose, un ester de cellulose et/ou des résines acryliques.

10. Comprimé multicouche selon la revendication 9, **caractérisé en ce que** l'on utilise l'éthylcellulose, l'hydroxypropylméthylcellulose, l'hydroxypropylcellulose, l'hydroxyméthylcellulose, l'acide poly(méth)acrylique et/ou leurs dérivés ainsi que leurs sels, amides ou esters.

11. Comprimé multicouche selon l'une quelconque ou plusieurs des revendications 7 à 9, **caractérisé en ce que** l'on utilise comme matériaux pour la matrice l'éthylcellulose, l'hydroxyéthylcellulose, l'hydroxypropylcellulose, l'hydroxypropylméthylcellulose, des mono- et diglycérides d'acides gras en C12-C30 et/ou des alcools gras en C12-C30 ou leurs mélanges.

12. Comprimé multicouche selon l'une quelconque ou plusieurs des revendications 1 à 11, **caractérisé en ce qu'**au moins une couche contient une forme particulaire ou à libération retardée de Tramadol ou Diclofénac et/ou de leurs sels correspondants physiologiquement compatibles, de préférence sous forme de granulés, de microcapsules et/ou de pastilles, de manière particulièrement préférée de pastilles fabriquées par extrusion et/ou sphéronisation.

13. Comprimé multicouche selon l'une quelconque ou plusieurs des revendications 1 à 12, **caractérisé en ce que** la couche de séparation présente une épaisseur de couche de 0,05 mm à 10 mm, de manière préférée de 0,1 mm à 5 mm et de manière particulièrement préférée de 0,15 mm à 3 mm.

14. Comprimé multicouche selon l'une quelconque ou plusieurs des revendications 1 à 13, **caractérisé en ce que** la couche de séparation se compose d'un matériau physiologiquement compatible.

15. Comprimé multicouche selon l'une quelconque ou plusieurs des revendications 1 à 14, **caractérisé en ce que** la couche de séparation est à base d'un matériau physiologiquement compatible peu perméable ou imperméable pour les principes actifs, de préférence un polymère, une cire, une matière grasse, un acide gras, un alcool gras ou un éther ou un ester correspondant et présente un point de fusion de ≥ 40°C.

16. Comprimé multicouche selon la revendication 15, **caractérisé en ce que** la couche de séparation est à base d'un polymère physiologiquement compatible, de préférence l'acétate de cellulose, l'éthylcellulose, le butyrate de cellulose, le polyéthylène ou des copolymères d'acétate d'éthylènevinyle.

17. Comprimé multicouche selon l'une quelconque ou plusieurs des revendications 1 à 14, **caractérisé en ce que** la couche de séparation se compose certes d'un matériau perméable en soi pour les principes actifs, l'épaisseur de la couche est cependant mesurée de telle sorte que les deux principes actifs n'entrent pas en contact l'un avec l'autre au cours de la durée de libération.

18. Comprimé multicouche selon la revendication 17, **caractérisé en ce que** la couche perméable est à base d'éthers de cellulose, d'esters de cellulose et/ou de résines acryliques, de préférence d'éthylcellulose, d'hydroxypropylcellulose, d'hydroxyméthylcellulose, d'hydroxyméthylpropylcellulose, d'acide poly(méth)acrylique et/ou leurs dérivés, leurs sels, amides ou esters.

19. Comprimé multicouche selon la revendication 17, **caractérisé en ce que** la couche de séparation perméable se compose d'hydroxyéthylcellulose, d'hydroxypropylcellulose, d'hydroxyméthylcellulose et/ou d'hydroxypropylméthylcellulose.

20. Comprimé multicouche selon l'une quelconque ou plusieurs des revendications 1 à 19, **caractérisé en ce que** la couche de séparation et/ou les couches contenant le principe actif contiennent d'autres additifs, tels que des agents de charge, des agents glissants, des lubrifiants et des agents de régulation de l'écoulement.

21. Comprimé multicouche selon l'une quelconque ou plusieurs des revendications 1 à 20, **caractérisé en ce qu'**il est pourvu d'au moins un enrobage.

22. Comprimé multicouche selon la revendication 21, **caractérisé en ce que** l'enrobage est à libération retardée.

23. Comprimé multicouche selon la revendication 22, **caractérisé en ce que** l'enrobage se compose d'un polymère non soluble dans l'eau ou d'une cire.

24. Comprimé multicouche selon la revendication 23, **caractérisé en ce que** l'on utilise comme polymère une résine polyacrylique ou un dérivé de cellulose, de préférence une alkylcellulose.

25. Comprimé multicouche selon la revendication 24, **caractérisé en ce que** l'on utilise comme polymères une éthylcellulose et/ou un poly(méth)acrylate.

26. Comprimé multicouche destiné à une administration biquotidienne selon l'une quelconque des revendications 1 à 25, **caractérisé en ce que** le Tramadol resp. le Diclofénac est libéré dans un intervalle de 8 heures à ≥ 70 % en poids resp. à ≥ 60 % en poids.

27. Comprimé multicouche destiné à une administration quotidienne unique selon l'une quelconque ou plusieurs des revendications 1 à 26, **caractérisé en ce que** le Tramadol resp. le Diclofénac est libéré dans un intervalle de 16 heures à ≥ 70 % en poids resp. à ≥ 60 % en poids.

28. Comprimé multicouche selon l'une quelconque ou plusieurs des revendications 1 à 27, **caractérisé en ce qu'**il présente au moins une couche de désagrégation, qui provoque lors d'un contact avec des liquides corporels aqueux la dissolution des différentes couches les unes par rapport aux autres.

29. Comprimé multicouche selon l'une quelconque ou plusieurs des revendications 1 à 28, **caractérisé en ce que** le comprimé contient au moins une barre de cassure qui permet de diviser la dose à administrer, de préférence de la diviser en deux.
